# EUROPEAN PATENT APPLICATION

(11) **EP 2 502 920 A1**
(43) Date of publication of application: **26.09.2012**
(21) Application number: 11159817.3
(22) Date of filing: 25.03.2011
(51) Int. Cl.: C07D 277/56

(54) **Crystallization process of Febuxostat from A**

(71) Applicant: SANDOZ AG, 4056 Basel (CH)
(72) Inventor: Hotter, Andreas, 6250, Kundl (AT); Adamer, Verena, 6230, Brixlegg (AT); Langes, Christoph, 6020, Innsbruck (AT)
(74) Representative: Prüfer & Partner GbR European Patent Attorneys

(57) **Abstract**

The present invention relates to a reliable and industrial applicable process for the preparation of crystalline 2-[3-Cyano-4-(2-methylpropoxy)phenyl]-4-methyl-5-thiazolecarboxylic acid polymorph A.

## Description

### Field of the Invention

The present invention relates to a reliable and industrial applicable process for the preparation of crystalline 2-[3-Cyano-4-(2-methylpropoxy)phenyl]-4-methyl-5-thiazolecarboxylic acid polymorph A.

### Background of the Invention

2-[3-Cyano-4-(2-methylpropoxy)phenyl]-4-methyl-5-thiazolecarboxylic acid, hereinafter designated as Febuxostat, regulates the biosynthesis of uric acid in vivo and is indicated for the use in the treatment of hyperuricemia and gout. It received marketing approval in EU (brand name Adenuric) and US (brand name Uloric) and is represented by the following general formula (I):

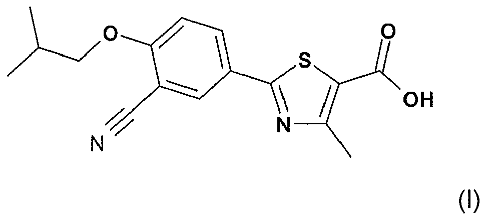

US 7,361,676 discloses formulations comprising form A.

EP1020454 discloses besides others polymorph A and also a method for the preparation of form A by crystallization from methanol/water. However, the preferred form A is difficult to make as form A is said to be obtainable in pure form only in a quite narrow window of temperature and methanol/water ratio in the region I as shown in figure 1 of EP1020454.

M. Kitamura, K. Nakamura, J. Crystal Growth, 236 (2002) 676-686 and M. Kitamura, M. Sugimoto, J. Crystal Growth, 237 (2003) 177-184 describe crystallizations of Febuxostat polymorphs from methanol/water systems and the influence of critical parameters such as crystallization temperature, methanol/water ratio, initial Febuxostat concentration and anti-solvent addition rate on the polymorphic form.

EP2039691 discloses a method of producing form A by crystallization from methanol/water within narrow optimized parameters in the presence of form A seed crystals in order to ensure the receipt of the desired polymorph A.

These processes are especially critical, as different polymorphic forms of Febuxostat are obtainable from the same solvent system methanol/water.

W02011/007895 A1 discloses a process for producing A-form crystals of Febuxostat comprising the step of heating and dissolving Febuxostat in one or more good solvents selected from a group consisting of 1-propanol, 2-propanol, ethanol and acetone, the step of cooling the solution, and the step of adding a hydrocarbon solvent as a poor solvent to the cooled solution. As poor hydrocarbon solvents heptane and hexane are mentioned.

The object of the present invention is to provide an improved process, which is in particular easy and reliable, for the preparation of polymorph A of Febuxostat suitable for industrial scale.

### Summary of the Invention

The present invention relates to a process for the preparation of Febuxostat polymorph A comprising the steps of:
a) dissolving Febuxostat in a mixture of n-propanol/n-heptane upon heating,
b) cooling the solution to obtain crystals, and
c) isolating the obtained crystals.

Preferably the isolated crystals are dried in a further step d).

The process according to the present invention, despite of being easy, is a reliable process for the preparation of polymorph A of Febuxostat suitable for industrial scale, and a relative high yield can be achieved.

The crystallization process of Febuxostat polymorph A from methanol/water as described in most part of the literature possesses several critical parameters such as crystallization temperature, methanol/water ratio, initial Febuxostat concentration and anti-solvent addition rate. These critical parameters are nonexistent in the process of the present invention.

Further, according to the present invention, polymorph A is obtained in a broad window of crystallization temperature and n-propanol/n-heptane ratio, without the need of seeding. As a general and significant advantage, the critical parameters of the anti-solvent addition timing and of anti-solvent addition rate are omitted, as the anti-solvent is already present in the starting dissolution step in the process of the present invention.

Furthermore the initial Febuxostat concentration of the process of the present invention does not influence the polymorphic form.

Overall, the present invention therefore provides a safe, easy, reliable and robust process for the preparation of polymorph A, especially suitable for industrial scale.

In addition the present invention refers to a pharmaceutical composition comprising crystalline form A prepared according to the process of the present invention and a pharmaceutically acceptable carrier.

Moreover the present invention relates to the use of crystalline form A prepared according to the process of the present invention for the production of a pharmaceutical composition.

Other objects, features, advantages and aspects of the present invention will become apparent to those of skill from the following description. It should be understood, however, that the description and the following specific examples, while indicating preferred embodiments of the invention, are given by way of illustration only. Various changes and modifications within the scope of the disclosed invention will become readily apparent to those skilled in the art from the description and from the other parts of the present disclosure.

Conventional Febuxostat polymorph A can be prepared by the process according to the present invention. In particular, Febuxostat polymorph A prepared according to the present invention is defined by X ray powder diffraction pattern having characteristic peaks at least at reflection angles 2 theta (also denoted "2 θ") of 6.6, 7.2, 12.9, 26.0, and 26.8, respectively ±0.2.Especially, Febuxostat polymorph A prepared according to the present invention is defined by X ray powder diffraction pattern having characteristic peaks at reflection angles 2 theta of 6.6, 7.2, 12.9, 13.3, 16.2, 16.6, 23.1, 23.9, 24.8, 26.0 and 26.8°, respectively ±0.2.

### Brief Description of the Drawings

Figure 1: XRPD spectrum of polymorph A of Febuxostat obtained from example 1 of the present invention
Figure 2: FTIR spectrum of polymorph A of Febuxostat obtained from example 1 of the present invention
Figure 3: TGA curve of polymorph A of Febuxostat obtained from example 1 of the present invention
Figure 4: Solubility and metastable limit curves of polymorph A of Febuxostat in n-propanol/n-heptane (1 : 3= v : v)

### Detailed Description of the Invention

The term "room temperature" as used herein indicates that the applied temperature is not critical and that no exact temperature value has to be kept. Usually, "room temperature" is understood to mean temperatures of about 15 °C to about 25 °C [see European Pharmacopoeia 7.0, 1.2 (2011)].

The present invention refers to a process for the preparation of Febuxostat polymorph A comprising the steps of:
a) dissolving Febuxostat in a mixture of n-propanol/n-heptane upon heating,
b) cooling the solution to obtain crystals,
c) isolating the obtained crystals, and optionally
d) drying the crystals.

Any form of Febuxostat may be applied in the above process. Suitable forms are e.g. amorphous Febuxostat, crystalline Febuxostat or mixtures thereof. Suitable crystalline forms are e.g. forms A, B, C, D or G of EP1020454 or forms F1 to F14 of W02010/144685.

Febuxostat is beneficially used at a concentration ranging from about 10 to 40 g/L, preferably from about 20 to 40 g/L, more preferably from about 30 to 40 g/L and most preferably the concentration used is about 40 g/L.

The Febuxostat starting material is in admixture with a solvent mixture comprising n-propanol and n-heptane. The n-propanol/n-heptane ratio ranges from about 1.0 : 0.5 to 1.0 : 3.0 (v : v), preferably from about 1.0 : 1.0 to 1.0 : 3.0 (v : v), more preferably from about 1.0 : higher than 2.0 to 1.0 : 3.0 (v :v) and most preferably the ratio is about 1.0 : 3.0 (v : v).

The dissolution temperature may range from about room temperature to about reflux temperature. However, the dissolution temperature depends on the n-propanol/n-heptane ratio and on the initial Febuxostat concentration. E.g. at a n-propanol/n-heptane ratio of about 1.0 : 3.0 (v : v) the temperature in the dissolution step may range from about 46 °C, when the applied Febuxostat concentration is about 10 g/L, to about 83 °C, when the applied Febuxostat concentration is about 40 g/L.

After receiving the solution the mixture is cooled to room temperature. During cooling the material crystallizes and a suspension is obtained. The cooling rate is not critical with respect to the polymorphic form and may preferably range from about -0.1 °C/min to -3.0 °C/min, more preferably from about -0.5 °C/min to -2.0 °C/min and most preferably the cooling rate is about -1 °C/min.

The obtained suspension is preferably further stirred at room temperature or below preferably for about 1 to 24 hours, more preferably for about 2 to 12 hours and most preferably for about 3 to 6 hours.

Afterwards the crystals are isolated, whereas any conventional method such as filtration, centrifugation or evaporation of the solvent may be applied.

Drying may be performed under vacuum at a temperature preferably ranging from about room temperature to about 80 °C, more preferably from about room temperature to about 60 °C and most preferably from about room temperature to about 50 °C. Drying is preferably conducted for about 1 to 72 hours, more preferably for about 1 to 48 hours and most preferably for about 1 to 24 hours.

Polymorph A obtained according to the process of the present invention shows a weight loss of less than about 0.50 weight%, preferably less than about 0.40 weight%, more preferably less than about 0.30 weight% and most preferably less than about 0.20 weight% in the range of about 30 to 200 °C determined by TGA.

The processes described in literature to obtain pure form A are especially critical, as different polymorphic forms of Febuxostat are basically obtainable, the result being not foreseeable. Several critical parameters such as crystallization temperature, solvent/anti-solvent ratio, initial Febuxostat concentration and anti-solvent addition time and addition rate may affect the polymorphic form.

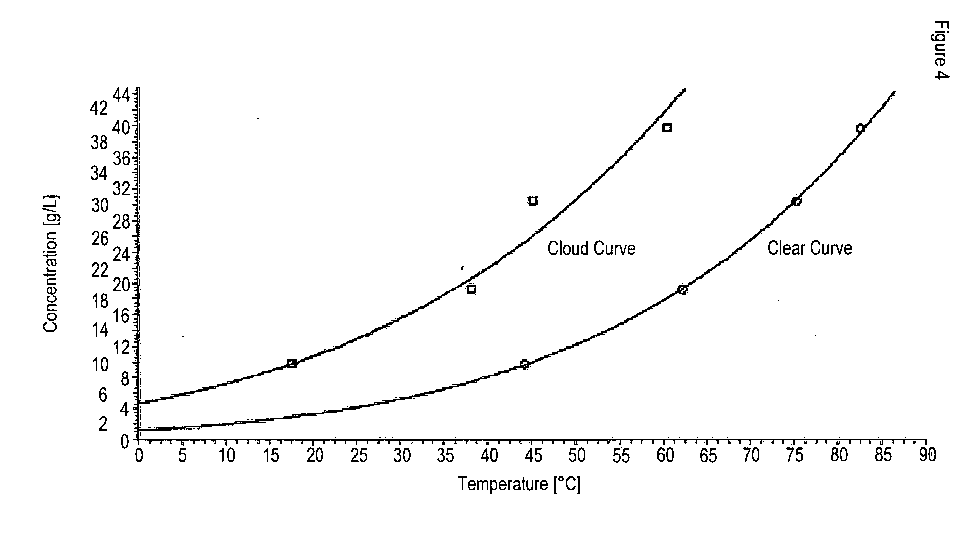

However, these critical parameters are nonexistent in the process of the present invention. For example, in the process of the present invention polymorph A is obtained in a broad window of crystallization temperature. As can be seen from figure 4 crystallization starts at about 60 °C when applying a Febuxostat concentration of about 40 g/L and at about 40 °C when the Febuxostat concentration is about 20 g/L. In both cases form A was obtained as shown in example 2 of the present invention. This is further indicative of the finding that the process according to the present invention is easy to perform, reliable and robust and suitable for industrial scale.

Polymorph A is obtained in a broad range of n-propanol/n-heptane ratio as well in the process of the present invention. Example 3 of the present invention shows that not the polymorphic form, but the yield is remarkably effected by an appropriate n-propanol/n-heptane ratio. Enhancement of yield becomes noticeable in particular when a more than twofold volume of n-heptane relative to n-propanol is applied in the mixture of n-propanol/n-heptane.

In addition in the process of the present invention the critical anti-solvent addition time point as well as the addition rate is omitted as the anti-solvent is already present in the dissolution step.

Furthermore the initial Febuxostat concentration of the process of the present invention does not influence the polymorphic form as can be seen from example 2, yet the yield can be enhanced especially when Febuxostat is dissolved in the mixture of n-propanol/n-heptane at a concentration ranging from 10 to 40 g/L, in particular when ranging from 20 to 40 g/L.

Example 4 of the present invention shows the independence of the polymorphic form on the cooling rate. Suitably, the cooling rate is set in the range of -1.0 °/min to -3.0 °/min, and preferably the cooling proceeds from a previous clear solution through a cloudy phase down to room temperature while agitating, and agitating is continued at room temperature for an appropriate further period.

In the present procedure only solvents with low toxic potential are used (class 3 solvents according to ICH guideline Q3C (R3) "Impurities: Guideline for residual solvents").

To sum it up the present invention provides a safe, easy, reliable and robust process for the preparation of polymorph A especially suitable for industrial scale.

The crystal form A prepared according to the process of the present invention may advantageously be employed in various pharmaceutical formulations for use in the treatment of hyperuricemia and gout and related diseases in accordance with the present invention. The present invention therefore also relates to a pharmaceutical composition which comprises the crystalline form A of Febuxostat prepared according to the process of the present invention and a pharmaceutically acceptable carrier.

The present invention therefore also relates to a pharmaceutical composition comprising the crystalline form A of Febuxostat prepared according to the process of the present invention, wherein form A is the only detectable crystalline form of Febuxostat, in particular the present invention relates to such pharmaceutical compositions, wherein more than 95 % of the crystalline form A present in said composition are stably present as form A.

"Stably present" as defined herein means that even after storage of the pharmaceutical composition for 180 days, and preferably even after storage for 2 years, the crystalline form of Febuxostat designated as form A initially comprised in the pharmaceutical composition is still present as crystalline form A after storage for the indicated period.

The pharmaceutical compositions of the invention comprising the crystalline form A of Febuxostat prepared according to the process of the present invention may further comprise one or more pharmaceutically acceptable excipients which are preferably selected from the group consisting of fillers, sweeteners, buffering agents, glidants, flowing agents, flavouring agents, lubricants, preservatives, surfactants, wetting agents, binders, disintegrants and thickeners. Other excipients known in the field of pharmaceutical compositions may also be used. Furthermore, the pharmaceutical composition may comprise a combination of two or more excipients also within one of the members of the above mentioned group.

Examples of suitable excipients for pharmaceutical compositions of the invention comprising Febuxostat are given in US2005/0043375A1, which is herein incorporated by reference, in paragraphs [0027] to [0030]. The excipients are typically contained in an amount of 50 to 98 parts by weight, and more preferably 60 to 95 parts by weight, based on 100 parts by weight of the solid preparation.

In paragraph [0028] US2005/0043375A1 discloses examples of the disintegrating agent for the pharmaceutical compositions of the present invention comprising Febuxostat. The disclosed disintegrants, which can also be used for the pharmaceutical composition of the present invention, include carmellose sodium, carmellose calcium, low-substituted hydroxypropyl cellulose, crosscarmellose sodium, carboxymethyl starch sodium and crosspovidone. Preferred disintegrants and preferred amounts for the disintegrating agent to be used in the pharmaceutical composition of the present invention are also disclosed in paragraph [0028] of US2005/0043375A1.

Paragraph [0029] of US2005/0043375A1 discloses examples of additional excipients to be added in the preparation of pharmaceutical compositions comprising Febuxostat, such as binders, lubricants, coating agents, plasticizers, diluents, colorants, preservatives, antiseptics or fragrance agents, which are also useful for the preparation of the pharmaceutical composition of the present invention.

In paragraph [0030] US2005/0043375A1 discloses examples of binders for the pharmaceutical composition of the present invention, such as hydroxypropyl cellulose, hydroxyl propylmethyl cellulose and polyvinyl pyrrolidone. The binder is contained in an amount of 0.5 to 25.0 parts by weight, preferably 1.0 to 20.0 parts by weight, based on 100 parts by weight of the pharmaceutical composition of the present invention.

Examples of suitable processes for the preparation of the pharmaceutical composition of the present invention are given in US2005/0043375A1, which is herein incorporated by reference, in paragraphs [0031] to [0033]. In summary the pharmaceutical compositions of the present invention are preferably solid preparations which can be produced by compressing a mixture of form A prepared according to the process of the present invention with excipients and disintegrating agents. For example, one method for the production of the pharmaceutical composition of the present invention includes mixing form A prepared according to the process of the present invention with suitable excipients in a suitable mixer. The mixture can then be directly compressed to tablets. Alternatively, a dry granulation step can be employed so as to produce granules suitable for tablet production. A wet granulation step can be employed to produce granules suitable for tablet production, in which step water, ethanol and solutions containing binders can be used.

Specific examples for the production of tablets of the present invention are given in US2005/0043375A1, paragraphs [0034] to [0048]. These examples can be repeated using form A prepared according to the process of the present invention.

The pharmaceutical compositions of the invention comprising the crystalline form A of Febuxostat prepared according to the process of the present invention are preferably packaged or filled into containers. Containers are typically used for stable storage of the pharmaceutical compositions of the invention, for example at room temperature for a prolonged period, e.g. for at least about 6 months, preferably at least about 24 months, e.g. for up to at least 24 months, e.g. for up to at least about 30 months, such as for up to about 60 months.

A preferred container is a bottle, in particular a glass bottle, having e.g. a screw closure, or is a blister, e.g. an aluminum blister or strip, e.g. a blister consisting of 2 aluminum foils or strips, or may be any other suitable container. More preferably said container is a gas-tight container, such as an air-tight container.

Preferred containers are glass bottles sealed with an aluminum membrane, alu-alu-blisters or strips. The container according to the invention is obtained by filling the pharmaceutical compositions of the invention into said container.

The present invention also relates to the use of crystalline form A prepared according to the process of the present invention for the production of a pharmaceutical composition, in particular a pharmaceutical composition intended for sale in a tropical country having areas with an Af or Am climate according to the Köppen-Geiger climate classification.

### Examples

The XRPD pattern was obtained with an X'Pert Pro diffractometer (PANalytical, Almelo, The Netherlands) equipped with a theta/theta coupled goniometer in transmission geometry, programmable XYZ stage with well plate holder, Cu-Kα_{1,2} radiation source (wavelength 0.15419 nm) with a focusing mirror, a 0.5° divergence slit, 0.02° soller slit collimator and a 1° anti-scattering slit on the incident beam side, a 2 mm anti-scattering slit, a 0.02° soller slit collimator and a Nickel filter on the diffracted beam side and a solid state PIXcel detector. The patterns were recorded at a tube voltage of 40 kV, tube current of 40 mA, applying a stepsize of 0.013° 2-theta with 80 s per step in the angular range of 2° to 40° 2-theta.

The IR spectrum was collected on a MKII Golden Gate^{™} Single Reflection Diamond ATR (attenuated total reflection) cell with a Bruker Tensor 27 FTIR spectrometer with 4 cm⁻¹ resolution at ambient conditions. To collect a spectrum a spatula tip of a sample was applied to the surface of the diamond in powder form. Then the sample was pressed onto the diamond with a sapphire anvil and the spectrum was recorded.

A spectrum of the clean diamond was used as background spectrum. A typical precision of the wavenumber values is in the range of about ± 2 cm⁻¹. Thus, an infrared peak that appears at 1716 cm⁻¹ can appear between 1714 and 1718 cm⁻¹ on most infrared spectrometers under standard conditions.

Thermogravimetric analysis was performed on a TGA/DSC 1 Stare-System (Mettler Toledo). 9.91 mg sample were heated in a pierced 100 µl Al-pan from 25 °C to 220 °C at a rate of 10 °C/min. Nitrogen was used as purge gas (sample purge: 50 mL/min, balance purge: 20 mL/min).

The solubility and metastable limit curves were detected with a Crystal16^{™} parallel crystallizer (Avantium Technologies). Febuxostat polymorph A was slurried in n-propanol/n-heptane (1 : 3 = v : v) at four different concentrations. The suspensions were dissolved upon heating (heating rate 1 °C/min) and recrystallized by cooling the obtained solutions (cooling rate -1 °C/min). The clear and cloud points detected by the Crystal16^{™} were used to create the solubility and metastable limit curves presented in figure 4 using Crystal Clear Version 1.0 software (Avantium Technologies) and Van't Hoff equation.

### Example 1: Preparation of Febuxostat polymorph A - best mode

A mixture of 3.20 g Febuxostat, 20 mL n-propanol and 60 mL n-heptane was heated to reflux. The obtained solution was cooled to room temperature whereas a white precipitate occurred during cooling. Then the suspension was further stirred at room temperature for 5 hours. Finally the solid was filtered off and dried for 16 hours at 40 °C under vacuum to obtain 2.74 g (86 % yield) of polymorph A.

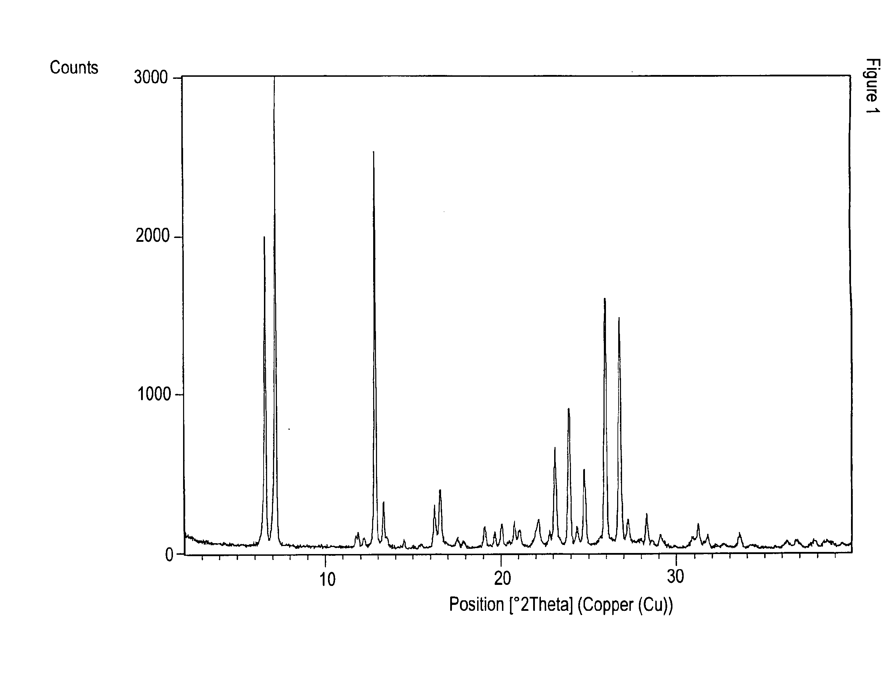

The receipt of polymorph A was confirmed by XRPD and the corresponding X-ray powder diffractogram is displayed in figure 1.

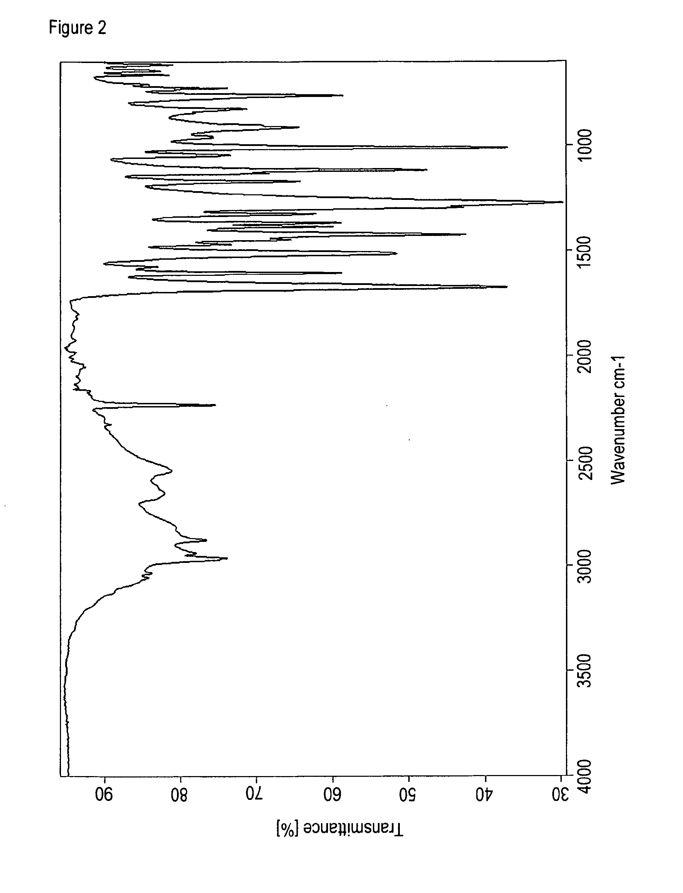

FTIR-spectroscopy also confirmed the presence of polymorph A and the corresponding spectrum is displayed in figure 2.

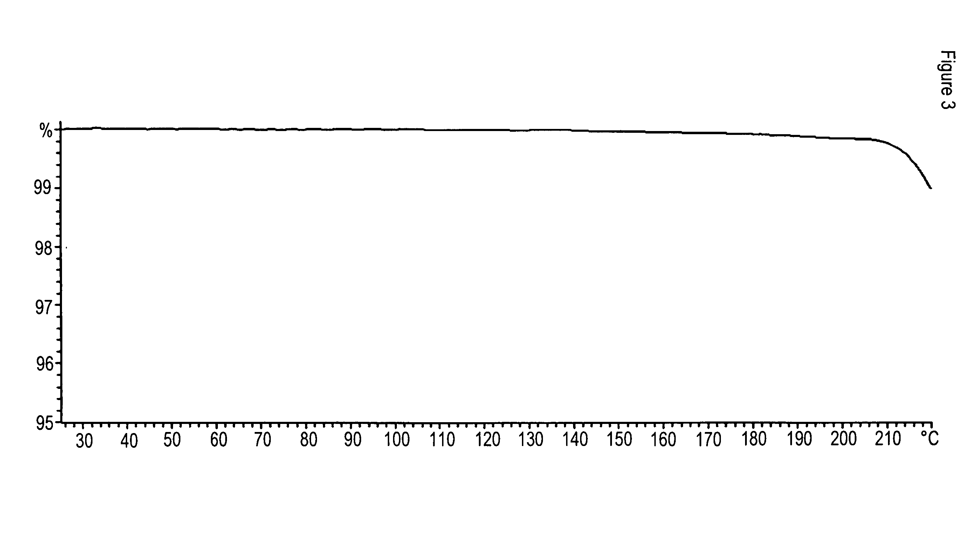

TGA shows a mass loss of about 0.16 weight% in the range from about 30 to 200 °C confirming the presence of an unsolvated respectively anhydrous form. The corresponding TGA-curve is displayed in figure 3.

### Example 2: Preparation of Febuxostat polymorph A - variable initial concentrations

The Febuxostat starting materials were dissolved in a mixture of n-propanol/n-heptane having a ratio of 1.0 : 3.0 (v : v) at concentrations according to table 1 upon heating. The obtained solutions were cooled to room temperature at a cooling rate of -1 °C/min, whereas crystallization occurred during cooling. The obtained suspensions were further stirred at room temperature for about 5 hours. The solid materials were isolated by filtration and dried under vacuum at 40 °C for about 16 hours to obtain Febuxostat polymorph A.

**Table 1: Variable initial concentrations**

| **Experiment** | **Concentration [g/L]** | **Yield [%]** | **Polymorph** |
|---|---|---|---|
| 1 | 20 | 71 | A |
| 2 | 40 | 86 | A |

### Example 3: Preparation of Febuxostat polymorph A - variable solvent ratio

The Febuxostat starting materials were dissolved in a mixture of n-propanol/n-heptane having a ratio as displayed in table 2 (40 g/L initial concentration) upon heating. The obtained solutions were cooled to room temperature at a cooling rate of -1 °C/min, whereas crystallization occurred during cooling. The obtained suspensions were further stirred at room temperature for about 5 hours. The solid materials were isolated by filtration and dried under vacuum at 40 °C for 16 hours to obtain Febuxostat polymorph A.

**Table 2: Variable solvent ratio**

| **Experiment** | **n-Propanol [ratio]** | **n-Heptane [ratio]** | **Yield [%]** | **Polymorph** |
|---|---|---|---|---|
| 1 | 1 | 1 | 72 | A |
| 2 | 1 | 3 | 86 | A |

### Example 4: Preparation of Febuxostat polymorph A - variable cooling rate

Febuxostat (40 g/L initial concentration) was dissolved in a mixture of n-propanol and n-heptane having a ratio of 1.0 : 3.0 (v : v) upon heating. The obtained solutions were cooled to room temperature at a cooling rate as displayed in table 3, whereas crystallization occurred during cooling. The obtained suspensions were further stirred at room temperature for 5 hours. The solid materials were isolated by filtration and dried under vacuum at room temperature for 16 hours to obtain Febuxostat polymorph A.

**Table 3: Variable cooling rate**

| **Experiment** | **Cooling rate [°C/min]** | **Yield [%]** | **Polymorph** |
|---|---|---|---|
| 1 | -1.0 | 86 | A |
| 2 | -3.0 | 83 | A |

## Claims

1. A process for the preparation of polymorphic pure form A of Febuxostat comprising the steps of:
a) dissolving Febuxostat in a mixture of n-propanol/n-heptane upon heating,
b) cooling the solution to obtain crystals, and
c) isolating the obtained crystals.

2. A process according to claim 1, wherein Febuxostat is dissolved in the mixture of n-propanol/n-heptane at a concentration ranging from 10 to 40 g/L.

3. A process according to claim 1, wherein Febuxostat is dissolved in the mixture of n-propanol/n-heptane at a concentration ranging from 20 to 40 g/L

4. The process according to any one of the preceding claims, wherein a more than twofold volume of n-heptane relative to n-propanol is applied in the mixture of n-propanol/n-heptane.

5. The process according to claim 4, wherein the n-propanol/n-heptane ratio is in the range of higher than 1.0 : 2.0 to 1.0 : 3.0 (v : v).

6. A process according to claim 4, wherein the n-propanol/n-heptane ratio is about 1 : 3 (v : v).

7. The process according to any one of the preceding claims, wherein the admixture of Febuxostat in n-propanol/n-heptane is heated in step a) to obtain a clear solution.

8. The process according to any one of the preceding claims, wherein the admixture of Febuxostat in n-propanol/n-heptane is heated to reflux in step a).

9. The process according to any one of the preceding claims, wherein no seed crystals are used during cooling step b) to obtain crystals.

10. The process according to any one of the preceding claims, wherein the cooling rate during cooling step b) is in the range of -0.1 °/min to -3.0 °/min.

11. The process according to any one of the preceding claims, wherein cooling in step b) is performed to room temperature while agitating, and agitating is continued at room temperature for further at least 1 hour, preferably at least 3 hours.

12. A process for the production of a pharmaceutical composition comprising polymorphic form A of Febuxostat, the process comprising the steps of:
preparing polymorphic form A of Febuxostat according to the process of any one of claims 1 to 11, and
mixing crystalline form A of Febuxostat with at least one pharmaceutically acceptable excipient.

13. The process according to claim 12, wherein more than 95 % of Febuxostat in the produced pharmaceutical composition is detectable as crystalline form A.

14. The process according to claim 13, wherein the more than 95 % of Febuxostat is still present after storage of the pharmaceutical composition for 180 days.

15. The process according to any one of claims 12 to 14, wherein the pharmaceutical composition is prepared as an oral dosage form.
